# EUROPEAN PATENT SPECIFICATION

(11) **EP 3 871 701 B1**
(45) Date of publication and mention of the grant of the patent: **30.07.2025**
(21) Application number: 19894768.1
(22) Date of filing: 22.11.2019
(51) Int. Cl.: A61L 9/14, B05B 7/24, B05B 12/12

(54) **CHEMICAL SOLUTION SPRAYING DEVICE**
SPRÜHVORRICHTUNG FÜR CHEMISCHE LÖSUNG
DISPOSITIF DE PULVÉRISATION DE SOLUTION CHIMIQUE

(30) Priority: 14.12.2018 JP 2018234864
(43) Date of publication of application: 01.09.2021
(73) Proprietor: DAIKIN INDUSTRIES, LTD., Osaka-Shi, Osaka 530-0001 (JP)
(72) Inventor: MIKI, Sanae, Osaka-shi, Osaka 530-8323 (JP)
(74) Representative: Goddar, Heinz J.
(86) International application number: PCT/JP2019/045811
(87) International publication number: WO 2020/121786

(56) References cited:
- WO-A1-2018/026932
- JP-A- 2002 355 643
- JP-A- 2004 141 618
- JP-A- 2004 162 511
- JP-A- 2014 013 107
- JP-A- 2017 003 190
- JP-A- 2017 003 190
- JP-A- 2017 009 243
- JP-A- 2017 169 613
- JP-A- 2018 166 945
- JP-A- 2018 166 945
- JP-A- H08 280 785
- JP-A- H09 103 476
- US-A- 4 184 612

## Description

### TECHNICAL FIELD

The present disclosure relates to a chemical solution spraying device.

### BACKGROUND ART

A spraying device according to Patent Document 1 supplies a scent material atomized by ultrasonic waves into a room.

JP 2017 003190 A and JP H08 280785 A disclose a chemical solution spraying device configured to supply a chemical solution to a target space in which an offensive odor is generated, a chemical solution spraying device comprising a sprayer configured to spray the chemical solution, a control unit configured to control at least one of start timing office spray operation of the sprayer or a spray amount at start of the spray operation of the sprayer based on a condition of the target space.

JP 2018 166945 A discloses a deodorizing system comprising a person detection unit and a controller. The operation mode is based on the detected number of people and their estimated activity using a correlation table.

US 4 184 612 A discloses a spraying device configured to be activated when someone enters the room.

JP 2017 169613 A relates to a robot for spraying a volume of liquid based on the detected malodor level.

### CITATION LIST

### PATENT DOCUMENT

Patent Document 1: Japanese Unexamined Patent Publication No. H04-309726

### SUMMARY OF THE INVENTION

### TECHNICAL PROBLEM

At the generation of an offensive odor in a target space of a spraying device, spraying a chemical solution (e.g., a scent material or a neutralizer) is conceivable so that a person in the space feels less unpleasant. On the other hand, in reducing the unpleasant feeling against the offensive odor, proper start timing of a spray operation or a proper spray amount at the start of the spray operation differs depending on the conditions (e.g., the concentration or offensive odor generation timing) of the target space. There has been no chemical solution spraying device that takes such conditions of the target space into consideration.

It is an objective of the present disclosure to provide a chemical solution spraying device capable of suitably controlling at least one of the start timing of a spray operation or a spray amount at the start of the spray operation.

### SOLUTION TO THE PROBLEM

A first aspect is directed to a chemical solution spraying device according to claim 1. The "chemical solution" used herein includes a "scent material" and a "neutralizer."

According to the first aspect, the control unit (60) controls at least one of the start timing of the spray operation or the spray amount at the start of the spray operation based on the condition of the target space (S).

A second aspect is an embodiment of the first aspect. In the second aspect, the chemical solution is a scent material.

According to the second aspect, the scent material as the chemical solution is sprayed from the sprayer (20). The scent material deodorizes the target space (S). The "deodorization" used herein includes "modulation" and "masking."

In the firs aspect, the chemical solution spraying device includes an offensive odor concentration sensor (50) configured to detect a concentration of the offensive odor in the target space (S), wherein the control unit (60) is configured to control start timing of a spray operation of the sprayer (20) and a spray amount at start of the spray operation of a sprayer (20) based on the condition of the target space (S).

In the first aspect, the chemical solution spraying device includes an entry detector (80) configured to detect an entry of a person into the target space (S).

According to the first aspect, when the concentration of the offensive odor detected by the offensive odor concentration sensor (50) is lower than the predetermined value and the entry detector (80) detects an entry of a person into the target space (S), the chemical solution spraying device starts the spray operation of the sprayer (20) in conjunction with such detection. A person who has just entered the space tends to feel unpleasant even if the concentration of the offensive odor is relatively low. The present invention reduces such unpleasant feeling of a person at the entry into the space.

### BRIEF DESCRIPTION OF THE DRAWINGS

FIG. 1 is a diagram illustrating a schematic overall configuration of a target space where a chemical solution spraying device according to an embodiment not belonging to the present invention is employed.
FIG. 2 is a diagram illustrating a schematic configuration of the chemical solution spraying device.
FIG. 3 is a block diagram of the chemical solution spraying device.
FIG. 4 is a time chart showing changes in the concentration of an offensive odor and the concentration of a chemical solution in the target space before and after the start of a spray operation of the chemical solution spraying device according to the embodiment not belonging to the present invention.
FIG. 5 is a diagram illustrating a schematic overall configuration of a target space where a chemical solution spraying device according to a first variation not belonging to the present invention is employed.
FIG. 6 is a time chart showing changes in the concentration of an offensive odor and the concentration of a chemical solution in the target space before and after the start of a spray operation of the chemical solution spraying device according to the first variation not belonging to the present invention.
FIG. 7 is a diagram illustrating a schematic overall configuration of a target space where a chemical solution spraying device according to a second variation not belonging to the present invention is employed.
FIG. 8 is a time chart showing changes in the concentration of an offensive odor and the concentration of a chemical solution in the target space before and after the start of a spray operation of the chemical solution spraying device according to the second variation not belonging to the present invention.
FIG. 9 is a diagram illustrating a schematic overall configuration of a target space where a chemical solution spraying device according to an embodiment of the present invention is employed.
FIG. 10 is a time chart showing changes in the concentration of an offensive odor and the concentration of a chemical solution in the target space before and after the start of a spray operation of the chemical solution spraying device according to the embodiment of the present invention.

### DESCRIPTION OF EMBODIMENT

An embodiment of the present invention will be described below with reference to the drawings. The embodiment below is merely exemplary one in nature, and is not intended to limit the scope, applications, or use of the present invention.

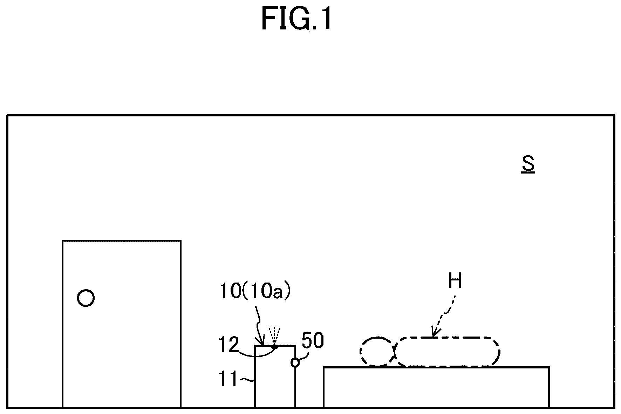

As shown in FIG. 1, a chemical solution spraying device (10) not belonging to the present invention is placed in an indoor space (S) of an elderly care facility, a hospital, or other places. In the indoor space (S), there is a person (H) who may emit a human waste smell that is an offensive odor. In the example of FIG. 1, the person (H) is the source of the offensive odor.

The chemical solution spraying device (10) sprays a chemical solution into the indoor space (S) which is a target space. The chemical solution of this example is a scent material containing a predetermined smell component. The scent material sprayed by the chemical solution spraying device (10) achieves deodorization. The "deodorization" used herein includes "modulation" and "masking." In the "modulation", the scent material is mixed into the offensive odor so that the mixed smell has a modified quality that is less perceivable as an offensive odor. In "masking", a relatively strong smell of a scent material reduces the sensitivity to the offensive odor. The chemical solution spraying device (10) according to this embodiment sprays a relatively small amount of a scent material corresponding to an offensive odor to modulate the offensive odor.

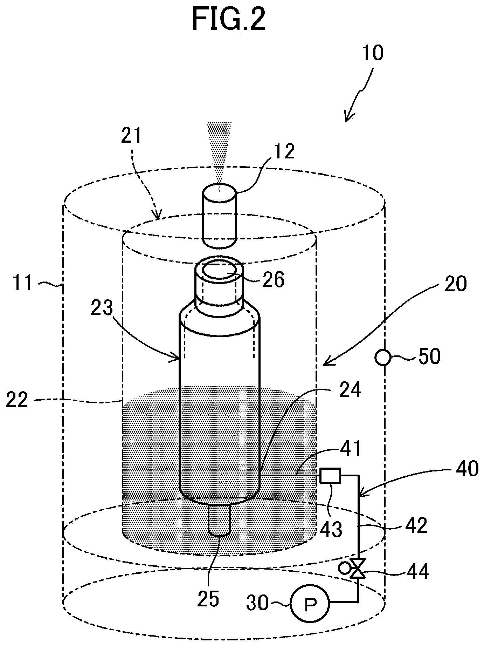

As shown in FIG. 2, the chemical solution spraying device (10) includes a spraying device body (10a) and an offensive odor concentration sensor (50) integrally attached to the spraying device body (10a). The spraying device body (10a) includes a casing (11), a spray unit (20) housed inside the casing (11), the offensive odor concentration sensor (50), and a control unit (60). The spray unit (20) constitutes a sprayer that sprays the chemical solution. The spray unit (20) includes a spray cartridge (21), an air pump (30), an air flow path (40), and an electromagnetic valve (31).

### <Casing>

The casing (11) is formed in a hollow. The casing (11) has a supply port (12) for supplying the atomized chemical solution to the indoor space (S).

### <Spray Unit>

The spray cartridge (21) is detachably attached to the inside of the casing (11). The spray cartridge (21) includes a tank (22) and a two-fluid nozzle (23). The tank (22) stores the chemical solution (i.e., the scent material). The two-fluid nozzle (23) constitutes an atomization mechanism that atomizes the chemical solution in the tank (22). Specifically, the two-fluid nozzle (23) atomizes the film of the chemical solution using the shearing force of the airflow. The atomization mechanism may be of another type such as a piezoelectric spraying type, an electrostatic spraying type, or an ultrasonic spraying type.

The two-fluid nozzle (23) has an inflow port (24) for letting the air in, a suction port (25) for sucking the chemical solution, and a spray port (26) for discharging the atomized chemical solution. The inflow port (24) is formed in the peripheral wall of the body of the two-fluid nozzle (23). The suction port (25) is formed at the lower end of the body of the two-fluid nozzle (23). The spray port (26) is formed at the upper end of the body of the two-fluid nozzle (23).

The air pump (30) has a discharger connected to the air flow path (40). The air pump (30) sucks the air in the indoor space (S) and discharges the sucked air to the air flow path (40). The air pump (30) supplies the air to the two-fluid nozzle (23) of the spray cartridge (21).

The air flow path (40) includes an inner flow path (41) and an outer flow path (42). The inner flow path (41) is located inside the spray cartridge (21). The outer flow path (42) is located outside the spray cartridge (21). The inner flow path (41) and the outer flow path (42) are connected to each other via a fluid joint (43). The fluid joint (43) is located outside the spray cartridge (21). The inner flow path (41) has an outflow end connected to the inflow port (24) of the two-fluid nozzle (23). The outer flow path (42) has an inflow end connected to the air pump (30).

The electromagnetic valve (31) is connected to the outer flow path (42). The electromagnetic valve (31) is an on-off valve that opens and closes the air flow path (40). Instead of the electromagnetic valve (31), a flow rate adjusting valve may be used which is capable of finely adjusting the opening degree of the air flow path (40).

### <Offensive Odor Concentration Sensor>

The offensive odor concentration sensor (50) detects the concentration of an offensive odor (a human waste smell in this example) generated in the indoor space (S). The offensive odor concentration used herein includes the concentration of the substance of the offensive odor, odor concentration of the offensive odor, and odor intensity corresponding to the concentration of the substance of the offensive odor. The offensive odor concentration sensor (50) according to this example detects the odor intensity of the offensive odor corresponding to the human waste smell. The offensive odor concentration sensor (50) according to this embodiment is integral with the spraying device body (10a) (see FIGS. 1 and 2). To be exact, the offensive odor concentration sensor (50) is provided in the casing (11) and exposed to the indoor space (S).

### <Control Unit>

The control unit (60) according to this embodiment controls the start timing of the spray operation of the spray unit (20) and the spray amount at the start of the spray operation, based on the conditions of the target space (indoor space (S)). In this embodiment, the concentration of the offensive odor in the target space is used as one of the "conditions of the target space".

The control unit (60) includes a microcomputer on a control board and a memory device (specifically, a semiconductor memory) storing software for operating the microcomputer.

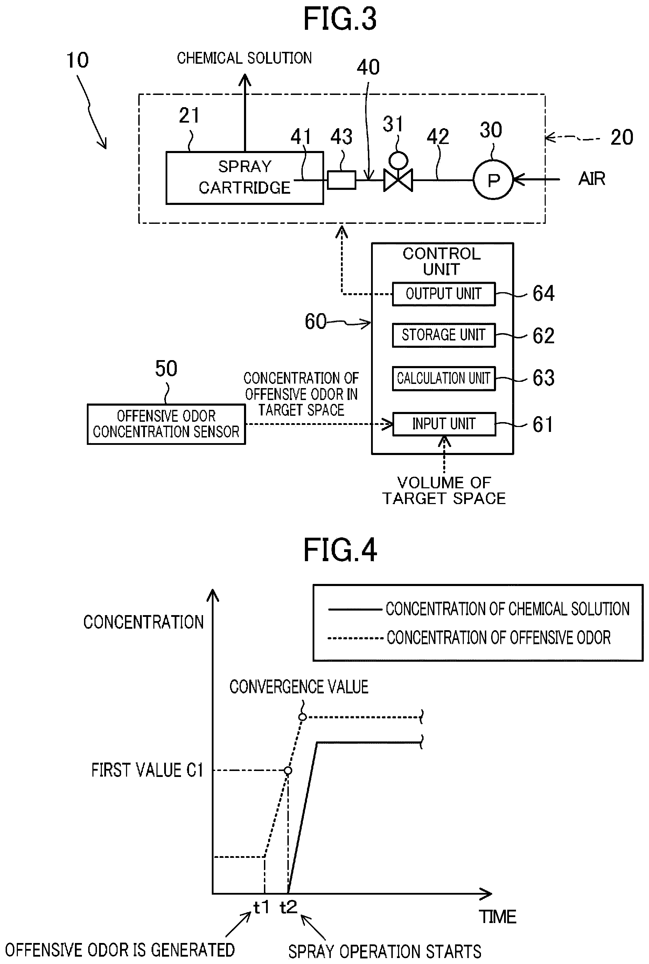

As shown in FIG. 3, the control unit (60) includes an input unit (61), a storage unit (62), a calculation unit (63), and an output unit (64). The input unit (61) receives signals indicating the concentration of the offensive odor detected by the offensive odor concentration sensor (50). The input unit (61) further receives the volume of the indoor space (S) that is a target space. The volume of the indoor space (S) can be set in advance by an operation unit (not shown). In addition, the input unit (61) may receive other indexes that influence the concentration of the offensive odor in the indoor space (S). These indexes include the amount of ventilation in the indoor space (S), the operating conditions of an air conditioner, the temperature and humidity of the air, and other characteristics.

The storage unit (62) stores at least data indicating the relationship between the concentration of the offensive odor in the target space and a proper concentration of the chemical solution corresponding to the concentration of the offensive odor. In addition, the storage unit (62) may store the volume of the space described above and other indexes.

The calculation unit (63) determines the start timing of the spray operation and the spray amount at the start of the spray operation. The calculation unit (63) according to this example determines whether or not the concentration of the offensive odor input to the input unit (61) exceeds a predetermined value (e.g., a first value (C1)). When the calculation unit (63) determines that the concentration of the offensive odor exceeds the first value (C1), the control unit (60) causes the output unit (64) to output a control signal for causing the spray unit (20) to start a spray operation. The start timing of the spray operation is controlled in this manner.

In addition, the calculation unit (63) obtains the concentration of the chemical solution required for deodorization based on the concentration of the offensive odor input to the input unit (61) and the volume of the space input to the input unit (61). The calculation unit (63) obtains the spray amount of the spray unit (20) to fill the indoor space (S) with this concentration of the chemical solution. The spray amount at the start of the spray operation is controlled in this manner. The calculation unit (63) according to this example predicts the convergence value of the concentration of the offensive odor in advance, based on a change in the concentration of the offensive odor from when the offensive odor has been generated to when the concentration of the offensive odor reaches the first value (C1). To be exact, the calculation unit (63) obtains the concentration of the chemical solution described above based on this convergence value of the concentration of the offensive odor.

When the concentration of the offensive odor exceeds the first value (C1) in the calculation unit (63), the output unit (64) outputs, to the spray unit (20), a control signal indicating a sufficient spray amount obtained by the calculation unit (63).

The spray amount at the start of the spray operation is adjusted by adjusting the time interval of the intermittent operation of the air pump (30), the time interval of the opening and closing operations of the electromagnetic valve (31), the discharge flow rate of the air pump (30), the opening degree of the air flow path (40), or other parameters. In addition, the chemical solution spraying device (10) configured to have a plurality of spray units (20) allows adjusting of the spray amounts according to the number of spray units (20) in operation.

### -Operation-

Details of the operation of the chemical solution spraying device (10) will be described with reference to FIGS. 1 to 4. When an offensive odor is generated in the indoor space (S), a person in the space feels unpleasant. In particular, immediately after the generation of the offensive odor, the nose of the person in the space is not used to the offensive odor, and tends to feel the offensive odor more unpleasant. In view of such a problem, the chemical solution spraying device (10) sprays the chemical solution at proper timing and a proper spray amount at the time of generation of an offensive odor.

As shown in FIG. 4, assume that a waste smell of a person (H) is generated as an offensive odor at a time point t1. The concentration of the offensive odor in the indoor space (S) gradually increases. For the sake of simplicity, FIG. 4 schematically shows changes in the concentration of the offensive odor and the concentration of the chemical solution. After the generation of the offensive odor, when the concentration of the offensive odor exceeds the first value C1 at a time point t2, a control signal is output from the output unit (64) of the control unit (60) to the spray unit (20) so that the chemical solution spraying device (10) performs a spray operation.

In the spray operation, the air pump (30) is turned on, and the electromagnetic valve (31) is open. The air transferred by the air pump (30) is introduced into the spray cartridge (21). The chemical solution is atomized in the two-fluid nozzle (23) by this air flow. The atomized chemical solution is supplied to the indoor space (S) through the spray port (26) and the supply port (12).

Immediately before the start of the spray operation, the calculation unit (63) obtains the proper concentration of the chemical solution for the indoor space (S). The chemical solution spraying device (10) according to this example modulates the offensive odor using a scent material. For this purpose, the calculation unit (63) calculates at which concentration of the chemical solution present in the indoor space (S) with respect to the concentration of the offensive odor, a sufficient modulation effect is obtainable, based on the data stored in the storage unit (62).

The calculation unit (63) obtains the concentration of the chemical solution using the convergence value of the concentration of the offensive odor after the generation of the offensive odor. The convergence value is predicted based on, for example, a change in the concentration of the offensive odor from the time point t1 when the offensive odor is generated to the time point t2 when the concentration of the offensive odor exceeds the first value (C1). The convergence value of the concentration of the offensive odor is obtainable in this manner at the time point t2 that is before the concentration of the offensive odor converges. It is therefore possible to determine, at the time point t2, the spray amount corresponding to the convergence value of the concentration of the offensive odor, and start the spray operation at the time point t2 at the determined spray amount. As a result, the spray operation can be started in advance before the converge of the concentration of the offensive odor to the maximum.

The control unit (60) may start the spray operation after a lapse of a predetermined delay time ΔT from the time point t2 at which the concentration of the offensive odor exceeds the first value (C1). In this case, the control unit (60) determines the proper concentration of the chemical solution, and hence the spray amount, based on the concentration of the offensive odor at the time after the lapse of ΔT from the time point t2.

In determining the spray amount based on the concentration of the chemical solution, the calculation unit (63) also uses the volume of the indoor space (S) and the other indexes described above.

Once the spray operation starts as described above, the concentration of the chemical solution in the indoor space (S) is adjusted to a proper concentration with respect to the concentration of the offensive odor. As a result, the scent material exhibits the sufficient modulation effect immediately after the generation of the offensive odor, making it possible to reduce unpleasant feeling of the person in the space. In addition, the adjustment reduces an excessive concentration of the chemical solution in the indoor space (S) with respect to the concentration of the offensive odor. Accordingly, a desired modulation effect is available and the consumption of the chemical solution can be reduced.

### -Advantages of Embodiment-

The chemical solution spraying device (10) according to the embodiment described above includes a spray unit (20) (sprayer) configured to spray a chemical solution, and a control unit (60) configured to control at least one of start timing of a spray operation of the spray unit (20) or a spray amount at start of the spray operation of the sprayer (20) based on a condition of an indoor space (S) (target space).

Specifically, the chemical solution spraying device (10) according to this embodiment includes an offensive odor concentration sensor (50) configured to detect a concentration of an offensive odor in the indoor space (S). The control unit (60) controls both of the start timing of the spray operation of the sprayer (20) and the spray amount at the start of the spray operation of the sprayer (20) based on the concentration of the offensive odor in the target space (S) detected by the offensive odor concentration sensor (50).

Immediately after the generation of the offensive odor in particular, a person in the space tends to feel unpleasant. The start timing of the chemical solution spray operation and the spray amount at the start of the spray operation are thus important to reduce the offensive odor. In this embodiment, the start timing of the spray operation and the spray amount at the start of the spray operation are controlled based on the concentration of the offensive odor detected by the offensive odor concentration sensor (50). It is therefore possible to start the spray operation at proper timing and at a proper spray amount, which reliably reduces the unpleasant feeling against the offensive odor.

The control unit (60) according to this embodiment starts the spray operation of the spray unit (20) in conjunction with an excess of the concentration of the offensive odor detected by the offensive odor concentration sensor (50) over a predetermined value (the first value (C1)). This operation supplies the chemical solution to the indoor space (S) at substantially the same timing as the generation and spread of the offensive odor in the indoor space (S), and quickly reduces the unpleasant feeling against the offensive odor.

The control unit (60) according to this embodiment sprays the chemical solution at the amount corresponding to the concentration of the offensive odor detected by the offensive odor concentration sensor (50), which reliably reduces the unpleasant feeling against the offensive odor and excessive consumption of the chemical solution.

In this embodiment, a scent material is used as the chemical solution sprayed from the spray unit (20) to modulate the offensive odor. Accordingly, the unpleasant feeling against the offensive odor can be reduced by simply spraying a relatively small amount of the scent material. It is therefore possible to reduce the consumption of the scent material in the spray cartridge (21) and the replacement frequency of the spray cartridge (21).

### <Variations of Embodiment>

The above embodiment may be implemented as the following variations.

### <First Variation>

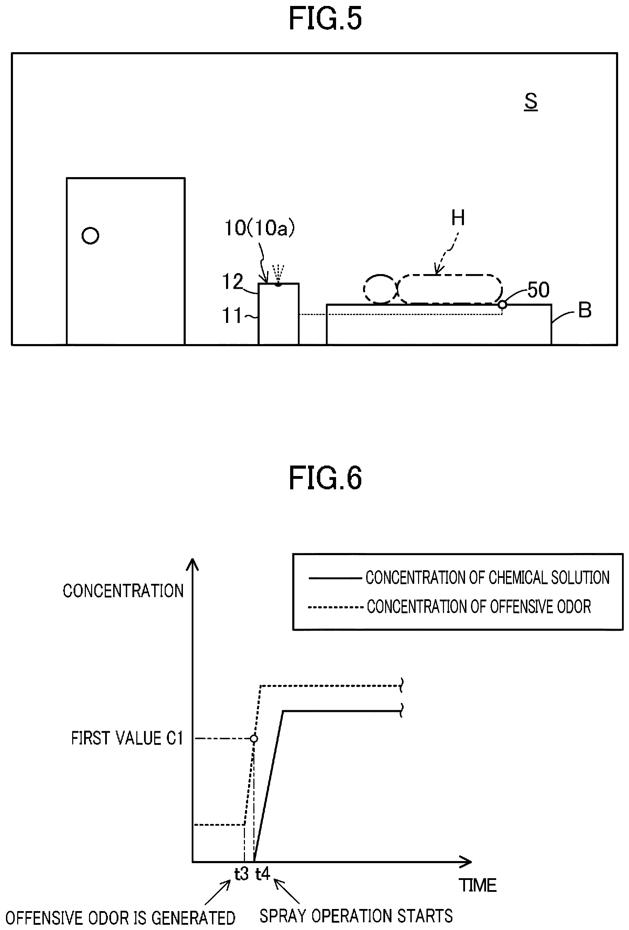

As shown in FIG. 5, in a chemical solution spraying device (10) according to a first variation not belonging to the present invention, an offensive odor concentration sensor (50) is an element independent of a spraying device body (10a). The offensive odor concentration sensor (50) is connected to an input unit (61) inside the spraying device body (10a) via a cable wire. The offensive odor concentration sensor (50) does not have to be wired to the input unit (61) but may be connected wirelessly.

The offensive odor concentration sensor (50) is located near the source (H) of an offensive odor. In this example, the waste smell of a person (H) is an offensive odor. That is, the offensive odor concentration sensor (50) is attached to, for example, a bed sheet (B). Other basic configurations in the first variation are the same as those in the embodiment described above.

As shown in FIG. 6, once an offensive odor is generated at a time point t3, the concentration of the offensive odor detected by the offensive odor concentration sensor (50) rapidly increases. This is because the offensive odor concentration sensor (50) is located near the source (H) of the offensive odor. In this example, since the concentration of the offensive odor exceeds the first value C1 earlier (i.e., at a time point t4 in FIG. 6) than in the embodiment described above, the spray operation starts immediately in conjunction with this excess. As a result, the chemical solution can be sprayed into the indoor space (S) before the offensive odor spreads in the indoor space (S).

The person in the space tends to feel more unpleasant if the offensive odor acts on the smell receptor of the person first. To avoid this, the first variation enables an immediate spray operation, which makes it possible for the scent material or a modulated smell to first act on the smell receptor. As a result, the unpleasant feeling of the person in the space can be reliably reduced.

### <Second Variation>

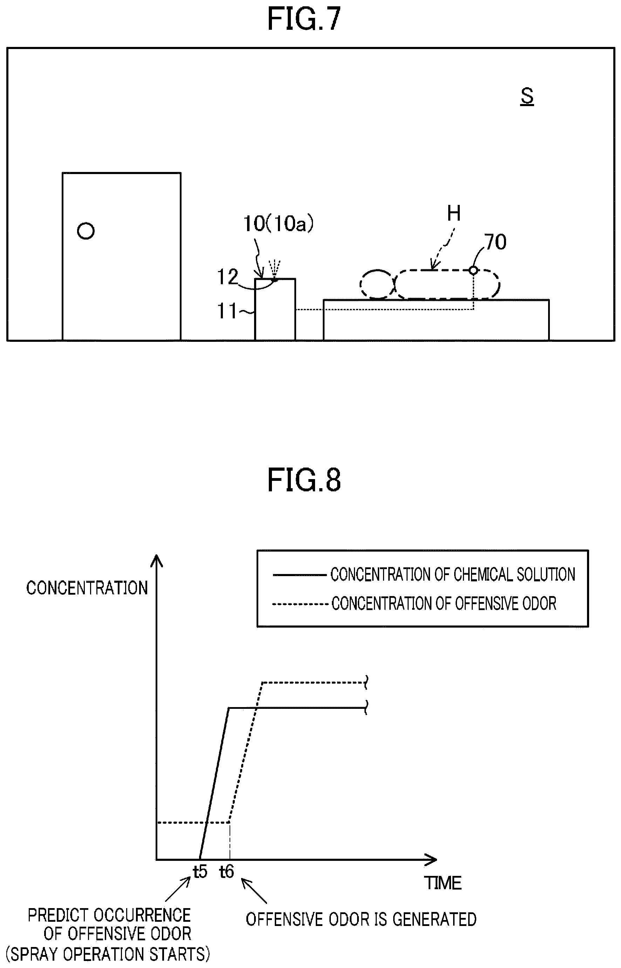

As shown in FIG. 7, a chemical solution spraying device (10) of a second variation not belonging to the present invention includes a body motion sensor (70). The body motion sensor (70) is attached near the urinary bladder or large intestine of a person (H), for example. The body motion sensor (70) detects the motion of the urinary bladder or large intestine using ultrasonic waves. The body motion sensor (70) detects the urination or defecation from this motion, and predicts the generation of a human waste smell in advance. The body motion sensor (70) constitutes a predictor configured to predict the generation of an offensive odor in advance.

As shown in FIG. 8, in the second variation, when the body motion sensor (70) predicts the generation of an offensive odor at a time point t5, a spray operation starts. The spray amount at this time is a predetermined amount set in advance with respect to the human waste smell. The human waste smell is generated thereafter at a time point t6.

The chemical solution (i.e., a scent material) has already been sprayed from the spray unit (20) at the time point t6. It is therefore possible to reliably reduce the unpleasant feeling against the offensive odor using the chemical solution. Thus, the second variation enables the scent material or a modulated smell to first act on the smell receptor of the person in the space. As a result, the unpleasant feeling of the person in the space can be reliably reduced.

Note that the predictor may be configured to predict the generation of an offensive odor due to the growth of bacteria, based on the temperature or humidity, for example. The predictor may be configured to predict the release (desorption) of the offensive odor adsorbed by a wall, based on the temperature and humidity.

### <Third Variation>

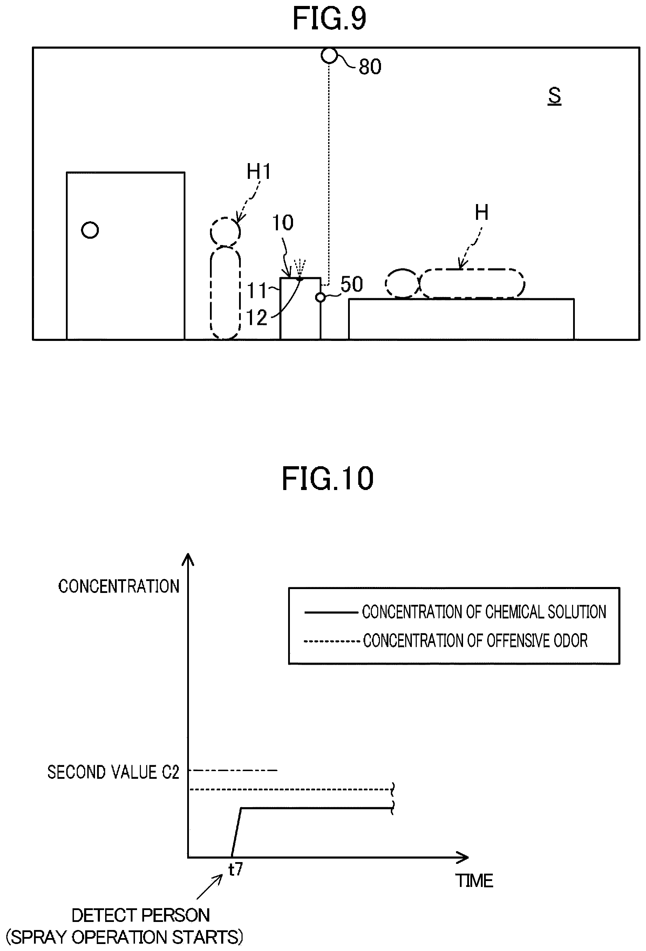

As shown in FIG. 9, a chemical solution spraying device (10) according to an embodiment of the present invention includes an entry sensor (80) configured to detect an entry of a person into an indoor space (S), and an offensive odor concentration sensor (50) that is the same as or similar to that in the embodiment described above. The entry sensor (80) includes an entry detector such as an infrared sensor, an optical sensor, an ultrasonic sensor, or a touch sensor attached to a door.

As shown in FIG. 10, assume that some degree of an offensive odor remains in the indoor space (S). In this case, the concentration of the offensive odor detected by the offensive odor concentration sensor (50) is a relatively low concentration. However, a person who has just entered the indoor space (S) tends to feel unpleasant even if the concentration of the offensive odor is relatively low. Hence, in this example, the spray unit (20) starts a spray operation when the concentration of the offensive odor detected by the offensive odor concentration sensor (50) is lower than a predetermined value (a second value) and the entry sensor (80) detects an entry of a person into the space. The spray amount at this time is a predetermined amount (a first spray amount) set in advance. As a result, the person who has entered the indoor space (S) feels less unpleasant against the offensive odor at a low concentration.

On the other hand, when the concentration of the offensive odor detected by the offensive odor concentration sensor (50) is higher than or equal to the predetermined value (the second value), the same control is performed as in the embodiment described above. That is, regardless of whether or not the entry sensor (80) has detected the entry of a person, the spray operation starts. The spray amount at this time may be determined based on the concentration of the offensive odor detected by the offensive odor concentration sensor (50). The spray amount at this time may be a predetermined amount (a second spray amount) larger than the first spray amount.

### «Other Embodiments»

The embodiment and variations described above may be implemented as follows.

The control unit (60) according to the embodiment described above may control only the spray amount at the start of the spray operation based on the conditions of the target space (S). Specifically, the start timing of the spray operation may be determined through manual operation, or a timer or other means, whereas the control unit (60) may control the spray amount at the start of the spray operation in accordance with the concentration of the offensive odor detected by the offensive odor concentration sensor (50).

The chemical solution sprayed from the spray unit (20) may be a neutralizer. For example, a slightly alkaline neutralizer may be sprayed against a slightly acidic offensive odor, or a slightly acidic neutralizer may be sprayed against a slightly alkaline offensive odor.

The target space (S) may be a space other than the indoor space as long as it is a space where an offensive odor may be generated and a person may be present.

The source of the offensive odor is not limited to a person and may be another organism (e.g., a dog, a cat, or other animals) that may emit a waste smell. The source of the offensive odor may be a diaper or a sheet containing excrement.

The offensive odor in the target space (S) may be human body odor. Examples of the body odor include sweat odor, fat odor, aging body odor, and foot odor. The offensive odor in the target space (S) may be a cigarette smell, a smoke smell during cooking, or other smells. Examples of the component of the offensive odor include at least one of isovaleric acid, 3-methyl-2-hexenoic acid, diacetyl, isovaleraldehyde, nonenal, 3-methyl-3-sulfanylhexan-1-ol, 3-hydroxy-3-methylhexanoic acid, hydrogen sulfide, methyl mercaptan, indole, pyridine, n-valeric acid, n-hexanal, or n-nonanal, or mixed components containing two or more thereof.

The chemical solution spraying device (10) may be of a floor mounted-type or any other types such as a wall-hanging type or a ceiling mounted type.

The chemical solution spraying device (10) may be mounted in an air treatment device configured to transfer air to the target space (S). The air treatment device includes an air conditioner, a ventilator, a humidity controller, and an air purifier.

The chemical solution spraying device (10) may be of the type configured to spray the chemical solution directly from the sprayer (20) into the target space (S), or may be of the type configured to supply air containing the sprayed chemical solution to the target space (S).

The storage unit (62) storing the relation between the concentration of the offensive odor and the concentration of the chemical solution may be located at the spray cartridge (21). In this case, the data related to the chemical solution corresponding to the spray cartridge (21) is stored in the storage unit (62). Accordingly, the data on the chemical solution stored in the storage unit (62) always matches the chemical solution in the spray cartridge (21). The storage unit (62) may be located in a server on a network to exchange data with the control unit (60).

While the embodiment and variations thereof have been described above, it will be understood that various changes in form and details may be made without departing from the scope of the claims. The embodiment, the variations, and the other embodiments may be combined and replaced with each other without deteriorating intended functions of the present disclosure. The expressions of "first," "second," and "third" described above are used to distinguish the terms to which these expressions are given, and do not limit the number and order of the terms.

### INDUSTRIAL APPLICABILITY

The present disclosure is useful in relation to a chemical solution spraying device.

### DESCRIPTION OF REFERENCE CHARACTERS

- 10: Spraying Device
- 10a: Spraying Device Body
- 20: Spray Unit (Sprayer)
- 50: Offensive Odor Concentration Sensor
- 60: Control Unit
- 70: Body Motion Sensor (Predictor)
- 80: Entry Sensor (Entry Detector)

## Claims

1. A chemical solution spraying device configured to supply a chemical solution to a target space (S) in which an offensive odor is generated, the chemical solution spraying device comprising:
a sprayer (20) configured to spray the chemical solution;
a control unit (60) configured to control start timing of a spray operation of the sprayer (20) and a spray amount at start of the spray operation of the sprayer (20) based on a condition of the target space (S);
an offensive odor concentration sensor (50) configured to detect a concentration of the offensive odor in the target space (S); and
an entry detector (80) configured to detect an entry of a person into the target space (S), **characterized in that**
the control unit (60) is configured to make the sprayer (20) spray a first spray amount of the chemical solution when a concentration of the offensive odor detected by the odor concentration sensor (50) is lower than a predetermined value and the entry detector (80) detects an entry of a person into the target space (S), and to make the sprayer (20) spray a second spray amount, which is larger than the first spray amount, of the chemical solution when the concentration of the offensive odor detected by the odor concentration sensor (50) is equal to or larger than the predetermined value.

2. The chemical solution spraying device of claim 1, wherein
the chemical solution is a scent material.

## Patentansprüche

1. Sprühvorrichtung für eine chemische Lösung, die konfiguriert ist, eine chemische Lösung einem Zielraum (S) zuzuführen, in dem ein unangenehmer Geruch erzeugt wird, wobei die Sprühvorrichtung für eine chemische Lösung Folgendes umfasst:
eine Sprüheinrichtung (20), die konfiguriert ist, die chemische Lösung zu sprühen;
eine Steuereinheit (60), die konfiguriert ist, einen Startzeitpunkt eines Sprühbetriebs der Sprüheinrichtung (20) und eine Sprühmenge zu Beginn des Sprühbetriebs der Sprüheinrichtung (20) basierend auf einem Zustand des Zielraums (S) zu steuern;
einen Konzentrationssensor (50) für einen unangenehmen Geruch, der konfiguriert ist, eine Konzentration des unangenehmen Geruchs in dem Zielraum (S) zu erfassen; und
einen Eintrittsdetektor (80), der konfiguriert ist, einen Eintritt einer Person in den Zielraum (S) zu erfassen, **dadurch gekennzeichnet, dass**
die Steuereinheit (60) konfiguriert ist, die Sprüheinrichtung (20) zu veranlassen, eine erste Sprühmenge der chemischen Lösung zu sprühen, wenn eine Konzentration des unangenehmen Geruchs, die durch den Geruchskonzentrationssensor (50) erfasst wird, kleiner als ein vorbestimmter Wert ist und der Eintrittsdetektor (80) einen Eintritt einer Person in den Zielraum (S) erfasst, und die Sprüheinrichtung (20) zu veranlassen, eine zweite Sprühmenge, die größer als die erste Sprühmenge ist, der chemischen Lösung zu sprühen, wenn die Konzentration des unangenehmen Geruchs, die durch den Geruchskonzentrationssensor (50) erfasst wird, gleich oder größer als der vorbestimmte Wert ist.

2. Sprühvorrichtung für eine chemische Lösung nach Anspruch 1, wobei
die chemische Lösung ein Duftstoffmaterial ist.

## Revendications

1. Dispositif de pulvérisation de solution chimique configuré pour fournir une solution chimique à un espace cible (S) dans lequel une odeur désagréable est générée, le dispositif de pulvérisation de solution chimique comprenant :
un pulvérisateur (20) configuré pour pulvériser la solution chimique ;
une unité de commande (60) configurée pour commander le moment de début d'une opération de pulvérisation du pulvérisateur (20) et une quantité de pulvérisation au début de l'opération de pulvérisation du pulvérisateur (20) sur la base d'une condition de l'espace cible (S) ;
un capteur de concentration d'odeur désagréable (50) configuré pour détecter une concentration de l'odeur désagréable dans l'espace cible (S) ; et
un détecteur d'entrée (80) configuré pour détecter l'entrée d'une personne dans l'espace cible (S),
**caractérisé en ce que**
l'unité de commande (60) est configurée pour que le pulvérisateur (20) pulvérise une première quantité de pulvérisation de la solution chimique quand une concentration de l'odeur désagréable détectée par le capteur de concentration d'odeur (50) est inférieure à une valeur prédéterminée et le détecteur d'entrée (80) détecte l'entrée d'une personne dans l'espace cible (S), et pour que le pulvérisateur (20) pulvérise une deuxième quantité de pulvérisation, qui est supérieure à la première quantité de pulvérisation, de la solution chimique quand la concentration de l'odeur désagréable détectée par le capteur de concentration d'odeur (50) est égale ou supérieure à la valeur prédéterminée.

2. Dispositif de pulvérisation de solution chimique selon la revendication 1, dans lequel
la solution chimique est une matière odoriférante.
